Europäisches Patentamt
European Patent Office
Office européen des brevets

(19) (11) Publication number: 0 110 496 B1

# (12) EUROPEAN PATENT SPECIFICATION

(45) Date of publication of patent specification: **02.09.87**

(21) Application number: **83304340.9**

(22) Date of filing: **27.07.83**

(51) Int. Cl.[4]: **C 07 D 215/20,** C 07 D 215/54, C 07 D 471/04 // A61K31/47 ,(C07D471/04, 231:00, 221:00)

## (54) Improvements in keto intermediates, their use and preparation.

(30) Priority: **03.11.82 US 438834**

(43) Date of publication of application: **13.06.84 Bulletin 84/24**

(45) Publication of the grant of the patent: **02.09.87 Bulletin 87/36**

(84) Designated Contracting States: **AT BE CH DE FR GB IT LI LU NL SE**

(56) References cited: **US-A-4 230 861**

(73) Proprietor: **ELI LILLY AND COMPANY**
**307, East McCarty Street**
**Indianapolis Indiana 46285 (US)**

(72) Inventor: **Schaus, John Mehnert**
**5427 North Delaware Street**
**Indianapolis Indiana 46220 (US)**

(74) Representative: **Crowther, Terence Roger et al**
**Erl Wood Manor**
**Windlesham Surrey GU20 6PH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

**Description**

This invention relates to novel keto intermediates, their preparation and their use in preparing other compounds.

A group of octahydropyrazolo[3,4-g]quinolines is disclosed in United States Patent 4,198,415 issued April 15, 1980, and in a divisional application thereof, United States Patent 4,230,861 issued October 28, 1980. Both intermediates and final products are disclosed therein and one reaction sequence so described is as follows:

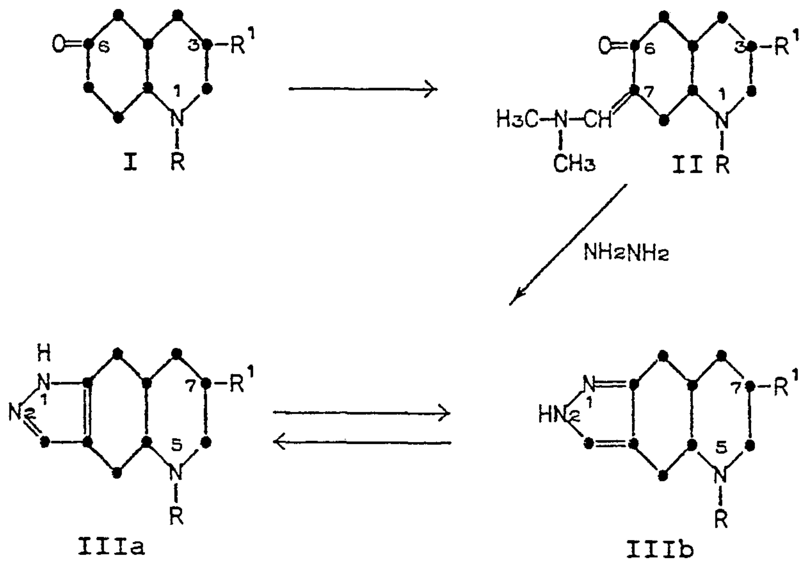


I

II

IIIa

IIIb

wherein R is H, $C_1$—$C_3$ alkyl, allyl or benzyl, $R^1$ is H or COOZ′ and Z′ is $C_1$—$C_2$ alkyl, benzyl, α-methylbenzyl, or phenylethyl. The compounds of formula IIIa or IIIb where $R^1$ is H and R is $C_1$—$C_3$ alkyl or allyl are useful as inhibitors of prolactin secretion and in the treatment of Parkinson's syndrome. The compounds of formula IIIa or IIIb where R and $R^1$ are H, where R is benzyl or where $R^1$ is COOZ′ are intermediates. The intermediates are converted by methods disclosed in the above patents to drugs. The reagent used to transform the 1-substituted-3-permissibly substituted-6-oxodecahydroquinoline (I) to the intermediate (II) is a dimethylformamide acetal such as dimethylformamide dimethylacetal.

Surprisingly, in accordance with this invention, it has been discovered that a novel keto intermediate provides a better process route to prepare the compounds of formulae IIIa and IIIb above. The advantages of using this novel intermediate are that cheaper reagents are used, that higher yields of the final product result, and that no isolation of the keto intermediate is required, although it may be isolated if desired.

Thus, in accordance with this invention, an improved method of preparing trans - dl - 5 - substituted - 7 - permissibly - substituted - 4,4a,5,6,7,8,8a,9 - octahydro - 1H(and 2H)pyrazolo - [3,4-g]quinolines of formulae IIIa and IIIb is set forth in Reaction Scheme I below:

Reaction Scheme I

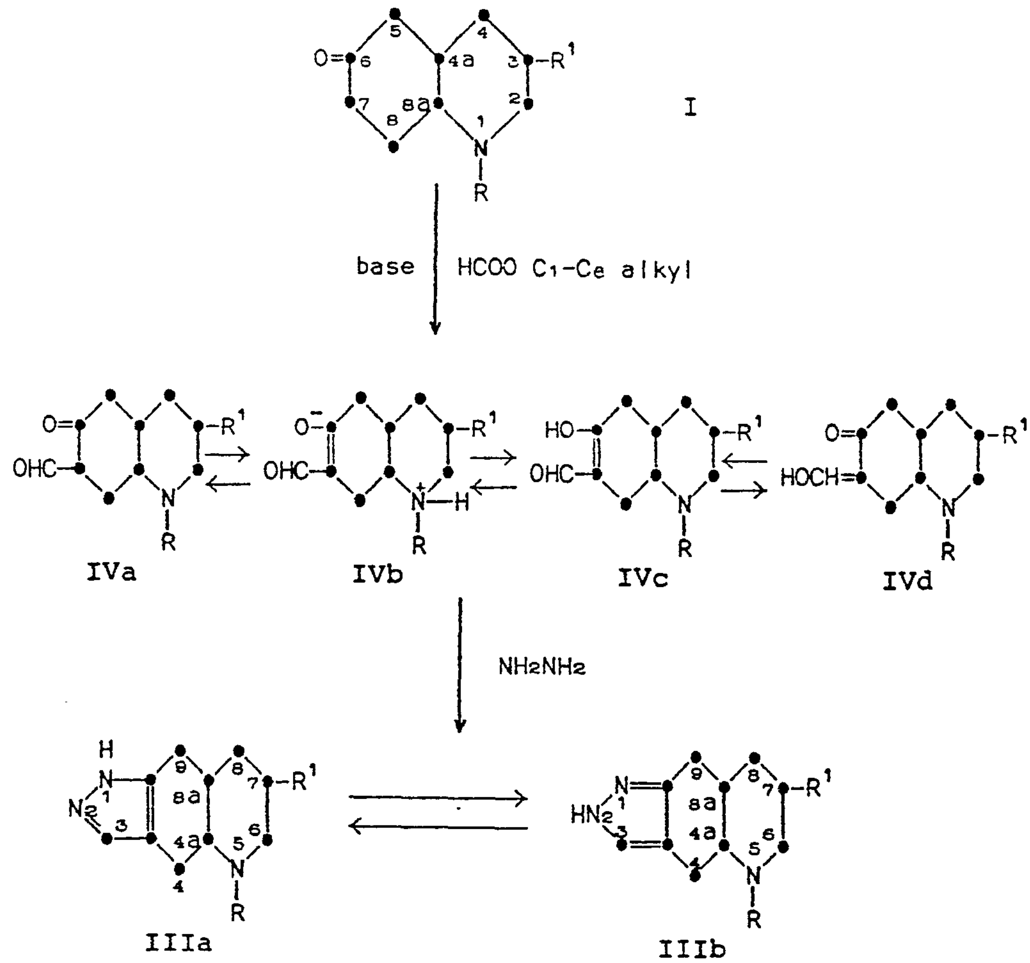


I

IVa IVb IVc IVd

IIIa IIIb

wherein R is $C_1$—$C_3$ alkyl, allyl or benzyl, $R^1$ is H or COOZ' and Z' is $C_1$—$C_2$ alkyl, benzyl, α-methylbenzyl, or phenylethyl.

According to Reaction Scheme I, a trans-dl-1-substituted-3-permissibly-substituted-6-oxodecahydroquinoline (I) is formylated with a $C_1$—$C_6$ alkyl formate in the presence of base to yield a trans-dl-1-substituted-3-permissibly-substituted-6-oxo-7-formyldecahydroquinoline, represented as a series of tautomeric structures (IVa-d). This intermediate is ordinarily not isolated and characterized as such but is reacted immediately *in situ* with hydrazine to yield as a mixture of tautomers-trans-dl-5-substituted-7-permissibly-substituted-4,4a,5,6,7,8,8a,9-octahydro-1H-pyrazolo[3,4-g]quinoline (IIIa) and trans-dl-5-substituted-7-permissibly-substituted-4,4a,5,6,7,8,8a,9-octahydro-2H-pyrazolo[3,4-g]quinoline (IIIb).

The formylated product, described above as four tautomeric structures (IVa-d), probably exists predominantly in aqueous solution as the zwitterion (IVb). However, all four tautomeric forms are in dynamic equilibrium, and in this specification, if any single structure is pictured or described, the other three are implied and contained herein. Also, resonance structures of various formulae above are also included, such as

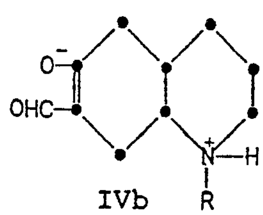

IVb

and

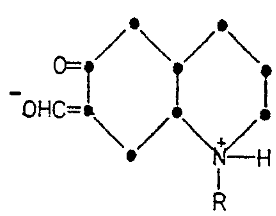

The first step of the above reaction is a modification of a Claissen condensation wherein a methylene group activated by an adjacent carbonyl group can be acylated in the presence of base. The base commonly employed is sodium ethylate. However, as will be apparent to those skilled in the art, other

bases such as the alkali metal *t*-alkoxides and hydrides, specifically, potassium *t*-butoxide, potassium *t*-amylalkoxide, or sodium hydride, can also be used. The Claissen condensation reaction (I → IV) is also usually carried out in ethanolic solution. As will also be apparent to those skilled in the art, other lower alkanols and similar polar anhydrous solvents can be employed as reaction media. Examples of suitable solvents are tetrahydrofuran (THF), diethyl ether, dimethoxyethane, dioxane, dimethylsulfoxide (DMSO), dimethylformamide (DMF) and *t*-butanol. Tetrahydrofuran is preferred as the solvent for the entire process in Reaction Scheme I. Although temperature of the reaction is not critical, a range from about −20°C. to reflux may be used, with 0°C. to room temperature being preferred.

In the ring closure step (IV → IIIa + IIIb), hydrazine is specified but hydrazine hydrate or salts of hydrazine can be used with equal success. Suitable solvents for the ring closure step are water, $C_1$—$C_4$ alkanols, especially *t*-butanol, THF, DMSO, dimethoxyethane, dioxane, and diethyl ether. The reaction can be run at a temperature from about 0°C. to reflux, with room temperature being preferred.

It is an advantage of the synthetic route described in Reaction Scheme I that both steps of the procedure can be carried out in the same reactor; i.e., it can be a "one-pot" process. Thus, solvents suitable for both reactions are preferred such as THF, DMSO, *t*-butanol, dimethoxyethane, diethyl ether, and dioxane, especially THF. If desired water or a $C_1$—$C_4$ alkanol can be added to the solvent system. A range of pH from about 13 to about 0 can be used, with a pH of about 9 being preferred. The preferred pH of about 9 is obtained by adding 10% HCl solution (1 mole) to the reaction mixture. Although a temperature range from about 0°C. to reflux can be used, room temperature is preferred.

A second advantage is that the yields of the pyrazole tautomers (IIIa + IIIb) are superior to those encountered with the process of the prior art which uses dimethylformamide dimethylacetal as a reagent. A further advantage is that the preferred formylating chemical employed, ethyl formate, is relatively inexpensive compared to dimethylformamide dimethylacetal.

It should be pointed out that the numbering of the ketone starting material (I) is different from that of the pyrazole final product (III). Thus, the asymmetric bridgehead carbon adjacent to the quinoline nitrogen is numbered 8a in the ketone while it is numbered 4a in the pyrazole. Furthermore, the other asymmetric bridgehead carbon is numbered 4a in the ketone while it is numbered 8a in the final product. The racemic pairs of formulae IIIa and IIIb are ordinarily referred to as a cis-dl pair and a trans-dl pair. The configuration of the molecule at C—4a and C—8a in the cis-dl pair would be a 4aR,8aS, and 4aS,8aR and for the trans-dl pair, 4aR,8aR, and 4aS,8aS. The starting chemical configurations are of course maintained in the synthesis of the pyrazoloquinoline since the Claissen condensation and subsequent ring closure with hydrazine do not affect configuration at these optical centers.

Our cofiled European Patent Application No. 112604 (Agents ref. X-6103) describes a method of separating trans-dl-l-n-propyl-6-oxodecahydroquinoline (I where R is n-propyl and R[1] is H) into its component stereoisomers Ia (4aR,8aR) and Ib (4aS,8aS).

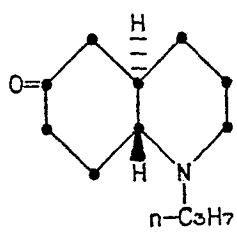

Ia

and

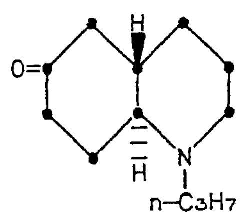

Ib

The procedure of the present invention is as applicable to the separated isomers (Ia and Ib) as it was to the trans-dl-racemate, shown in Reaction Scheme I.

The sequence of reactions from Reaction Scheme I is repeated in Reaction Sequence II below using the desired isomer, 4aR,8aR-1-n-propyl-6-oxodecahydroquinoline, to prepare the desired 4aR,8aR-4,4a,5,6,7,8,8a,9-octahydro-1H(and 2H)pyrazolo[3,4-g]quinoline.

Reaction Sequence II

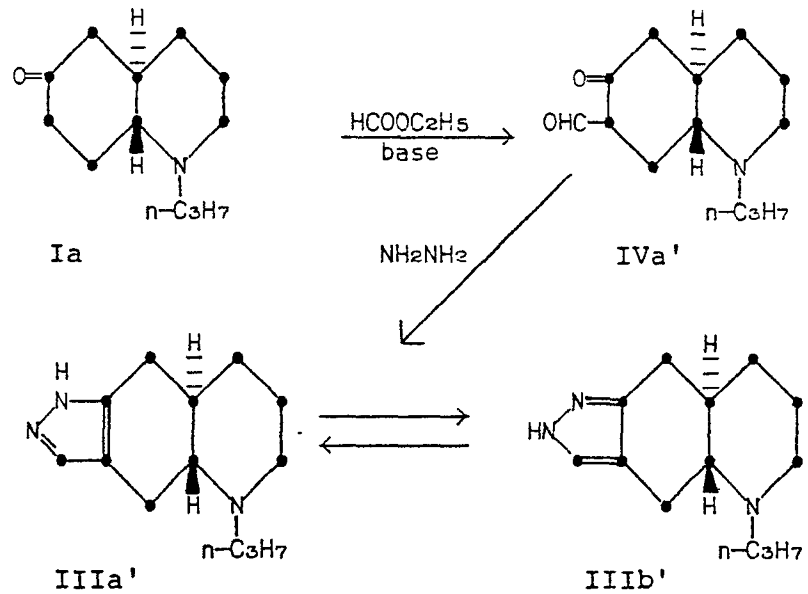


Ia IVa' IIIa' IIb'

An advantage of the process set forth in Reaction Scheme II lies in the fact that the trans-dl-ketone (I) is resolved and the pure 4aR,8aR stereoisomer (Ia) cyclized to yield an optically-active trans-4aR,8aR-octahydropyrazolo[3,4-g]quinoline, rather than cyclizing the trans-dl-racemate and resolving the trans-dl-pyrazoloquinoline. Since one-half at least of a racemic mixture is discarded during a resolution, it is clearly more economical to discard half of a starting material than half of a final product, particularly since organic reactions such as the cyclization of the ketonquinoline to a pyrazoloquinoline are never quantitative.

Particular processes are those which comprise (a) reacting a 4aR,8aR-1-$C_1$—$C_3$ alkyl-6-oxodecahydroquinoline with a $C_1$—$C_6$ alkyl formate in the presence of base to form a 4aR,8aR-1-$C_1$—$C_3$ alkyl-6-oxo-7-formyldecahydroquinoline, and then reacting said formyl compound with hydrazine to form the tautomeric mixture 4aR,8aR-5-$C_1$—$C_3$ alkyl-4,4a,5,6,7,8,8a,9-octahydro-1H(and 2H)pyrazolo[3,4-g]-quinoline.

In the following specific examples, which illustrate the present invention, the listed abbreviations are used.

THF = tetrahydrofuran
tlc = thin-layer chromatography

Example 1

One and one-half g. of potassium *t*-butoxide was weighed into a dry 250 ml. round-bottom flask. Twenty-five ml. of THF were added to dissolve the potassium *t*-butoxide. Next, a solution containing 0.81 ml. of ethyl formate, 0.97 g. of trans-dl-1-n-propyl-6-oxodecahydroquinoline and 10 ml. of THF were added to the butoxide solution. The reaction mixture was maintained at ambient temperature for about 45 minutes. Two ml. of hydrazine were then added followed by sufficient 15% aqueous hydrochloric acid to lower the pH to about 9. The consequent reaction mixture was stirred for 30 minutes at ambient temperature at which time tlc indicated that no ketone starting material was present. The reaction mixture was then poured into dilute (10%) aqueous sodium hydroxide and the alkaline mixture extracted with methylene dichloride (equal volume). The extract was dried and the solvent removed by evaporation *in vacuo* to yield 1.31 g. of a yellow oil comprising crude trans-dl-5-n-propyl-4,4a,5,6,7,8,8a,9-octahydro-1H(and 2H)pyrazolo[3,4-g]quinoline.

Example 2

A sufficient amount of 55% suspension of sodium hydride in mineral oil to yield 360 mg. (15 mmoles) of sodium hydride was placed in a 25 ml. round-buttom flask. The mineral oil was removed from the sodium hydride by washing three times with hexane. The residual sodium hydride was then suspended in 6 ml. of THF. Ethyl formate (740 mg.) plus one drop of anhydrous ethanol were next added followed by 975 mg. of trans-dl-1-n-propyl-6-oxodecahydroquinoline in 4 ml. of THF. The reaction mixture, which began to reflux almost immediately, was maintained at reflux temperature for about 45 minutes after which time tlc showed no remaining starting material. 50 ml. of water and 4 ml. of hydrazine were added and the pH adjusted with dilute aqueous hydrochloric acid to about pH=9. This reaction mixture was stirred at ambient temperature over night, forming a precipitate. The precipitate consisting of 373 mg. of a white powder,

trans-dl-5-n-propyl-4,4a,5,6,7,8,8a,9-octahydro-1H(and 2H)pyrazolo[3,4-g]quinoline, melting at 78—84°C. was collected.

Analysis:
Calculated for $C_{13}H_{21}N_3$:
C, 71.19; H, 9.65; N, 19.16
Found: C, 70.89; H, 9.15; N, 19.34.

Additional material was obtained by pouring the filtrate into dilute aqueous sodium hydroxide solution and extracting this alkaline solution with several portions of methylene dichloride. Concentration of the combined methylene dichloride extracts after drying yielded an additional 623 mg. of a white foam which was purified by chromatography over silica using THF containing a trace of aqueous ammonium hydroxide as the eluant. Early fractions shown to contain the desired pyrazolo[3,4-g]quinoline were combined to yield, after evaporation of the solvent, 437 mg. of a colorless oil.

This oil was converted to the dihydrochloride salt which melted at about 252—263°C. after recrystallization from a methanol/acetone solvent mixture.

The above run was repeated except that 125 mg. of trans-4aR,8aR-n-propyl-6-oxodecahydroquinoline were used and the amounts of ethyl formate and base (sodium hydride in place of potassium *t*-butoxide) decreased proportionately. After the reaction was substantially complete, as shown by lack of starting material on tlc analysis, the reaction mixture was poured into dilute aqueous sodium hydroxide and the alkaline mixture extracted with methylene dichloride. Drying of the methylene dichloride extract followed by removal of the solvent *in vacuo* yielded about 144 mg. of a colorless viscous oil which yielded only a single spot on tlc. The oil was dissolved in methanol and 0.20 N aqueous hydrochloric acid added (3.2 ml.). Concentration of the resulting yellow solution yielded a yellow semi-solid material which was dissolved in methanol. The methanol solution was decolorized with carbon and the carbon removed by filtration through Celite®. Evaporation of the solvent and recrystallization of the resulting residue from a methanol/ethyl acetate solvent mixture gave 80 mg. of a butter yellow powder — 4aR,8aR-5-n-propyl-4,4a,5,6,7,8,8a,9-octahydro-1H(and 2H)pyrazolo[3,4-g]quinoline; $[\alpha]_D^{25} = -121.76°$. ($H_2O$, c = 1).

## Example 3

A solution of 52 g. of optically pure 4aR,8aR-1-n-propyl-6-oxodecahydroquinoline and 79 g. of ethyl formate in 250 ml. of THF was added to a solution of 59.8 g. of potassium *t*-butoxide in 600 ml. of THF previously cooled to about 0°C. Gas evolved during the addition. The reaction mixture was stirred at about 0°C. for one-half hour and at ambient temperature for an additional hour. Twenty-five and six tenths grams of hydrazine were added and the pH of the solution adjusted to pH~9 with 10% aqueous hydrochloric acid (about 500 ml.). This reaction mixture was stirred vigorously at ambient temperature for two hours, after which time it was poured into water. The aqueous mixture was made strongly basic (pH~13) with dilute aqueous sodium hydroxide. The alkaline mixture was extracted with methylene dichloride, and the methylene dichloride extract separated and dried. Evaporation of the solvent left a yellow foam as a residue which by tlc contained the desired pyrazoloquinoline plus a minor amount of a single impurity. The residue was dissolved in 1 liter of hot methanol to which was added 250 ml. of 1N aqueous hydrochloric acid. Concentration of the solution yielded 65.4 g. of 4aR,8aR-5-n-propyl-4,4a,5,6,7,8,8a,9-octahydro-1H(and 2H)-pyrazolo[3,4-g]quinoline hydrochloride as a light yellow solid. Recrystallization from methanol/ethyl acetate gave 51.7 g. (76% yield) of a slightly yellow granular solid; $[\alpha]_D^{25} = -121.0°$ ($H_2O$, c = 1; $[\alpha]_{365}^{25} = -377.40$ ($H_2O$, c = 1).

Analysis
Calculated: C, 61.04; H, 8.67; N, 16.43; Cl, 13.86
Found: C, 61.32; H, 8.53; N, 16.22; Cl, 14.08

## Example 4

To a solution of 3.6 g. of potassium *t*-butoxide in 50 ml. THF at 0°C. was added a solution containing 5.0 g. of trans-dl-1-n-propyl-6-oxodecahydroquinoline and 2.36 g. of ethyl formate in 25 ml. of THF. The solution was stirred at 0°C. for 15 minutes and then at room temperature for 17 hours. A yellow precipitate formed which was collected by vacuum filtration, washed with THF, and dried under vacuum to yield the potassium salt of trans-dl-1-n-propyl-6-oxo-7-formyldecahydroquinoline. NMR ($D_2O$): δ 9.00 (s, 1H, CHO), δ 3.02—0.99 (m, 16H), δ 0.84 (t, J = 7, 3H, —$CH_3$ of n-$C_3H_7$).

The above 7-formyl product can be further reacted to prepare the compounds of formula IIIa and IIIb by methods disclosed above.

**Claims for the Contracting States: BE CH DE FR GB IT LI LU NL SE**

1. A compound of the formula

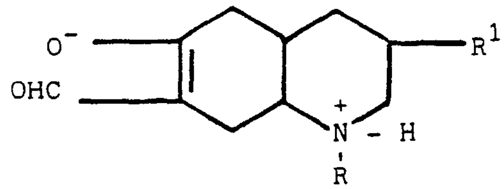

IVb

and its tautomers, where R is $C_1$—$C_3$ alkyl, allyl or benzyl and $R^1$ is H or COOZ′ and Z′ is $C_1$—$C_2$ alkyl, benzyl, α-methylbenzyl, or phenylethyl.

2. A compound of claim 1 in which R is n-propyl and $R^1$ is H and its tautomers.

3. 4aR,8aR-1-n-Propyl-6-oxo-7-formyldecahydroquinoline and its tautomers.

4. 4aS,8aS-1-n-Propyl-6-oxo-7-formyldecahydroquinoline and its tautomers.

5. trans-dl-1-n-Propyl-6-oxo-7-formyldecahydroquinoline and its tautomers.

6. A process for reacting a compound of formula IVb as defined in claim 1 and its tautomers, with hydrazine to yield a tautomer of formula IIIa or IIIb of the formula:

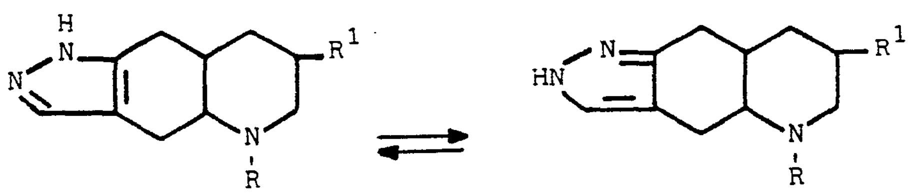

IIIa IIIb

when R and $R^1$ are defined as in claim 1.

7. A process of claim 6 in which $R^1$ is H and the ketone starting material is a trans-dl racemate.

8. A process of claim 6 in which the ketone starting material has the 4aR,8aR configuration.

9. A process of claim 6 in which the ketone starting material has the 4aS,8aS configuration.

10. The process which comprises reacting a decahydroquinoline of the formula

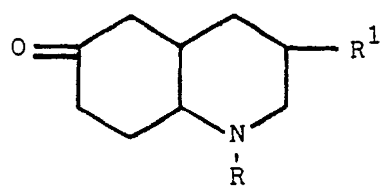

I

wherein R is $C_1$—$C_3$ alkyl, allyl or benzyl and $R^1$ is H or COOZ′ wherein Z′ is $C_1$—$C_2$ alkyl, benzyl, α-methylbenzyl, or phenylethyl, with a $C_1$—$C_6$ alkyl formate in the presence of base to yield a formyl derivative of the formula

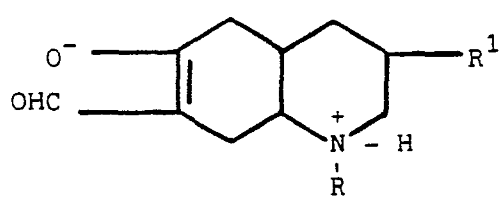

IVb

wherein R and $R^1$ have their previous significance.

11. A process of claim 10 wherein R is n-propyl and $R^1$ is H.

12. A process of claim 10 in which the 1-n-propyl-6-oxodecahydroquinoline starting material is the 4aR,8aR enantiomer.

13. A process of claim 10 in which the 1-n-propyl-6-oxodecahydroquinoline starting material is the 4aS,8aS enantiomer.

14. A process of claim 10 wherein the derivative of formula IVb is further reacted *in situ* with hydrazine according to the process of claim 6.

15. The process of claim 14 where $R^1$ is H and R is n-propyl.

16. The process of claim 14 or 15 wherein the solvent is tetrahydrofuran and the $C_1$—$C_6$ alkylformate is ethyl formate.

17. The process of claim 16 where the compound of formula I is the 4aR,8aR-1-n-propyl-6-oxodecahydroquinoline.

18. The process of claims 14 to 16 where the compound of formula I is the 4aS,8aS-1-n-propyl-6-oxodecahydroquinoline.

19. The process of claim 14 which comprises reacting a 4aR,8aR-1-$C_1$—$C_3$ alkyl-6-oxodecahydroquinoline with a $C_1$—$C_6$ alkyl formate in the presence of base to form a 4aR,8aR-1-$C_1$—$C_3$ alkyl-6-oxo-7-formyldecahydroquinoline, and then reacting said formyl compound with hydrazine to form the tautomeric mixture 4aR,8aR-5-$C_1$—$C_3$ alkyl-4,4a,5,6,7,8,8a,9-octahydro-1H(and 2H)pyrazolo[3,4-g]-quinoline.

20. A process of claim 19 in which 4aR,8aR-1-n-propyl-6-oxodecahydroquinoline is converted to the tautomeric mixture 4aR,8aR-5-n-propyl-4,4a,5,6,7,8,8a,9-octahydro-1H(and 2H)pyrazolo[3,4-g]quinoline.

**Claims for the Contracting State: AT**

1. A process for reacting a compound of the formula

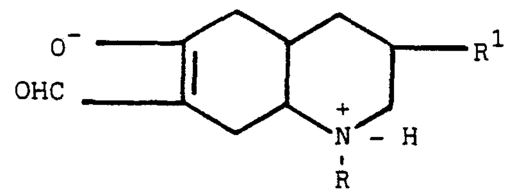

IVb

and its tautomers, where R is $C_1$—$C_3$ alkyl, allyl or benzyl and $R^1$ is H or COOZ' and Z' is $C_1$—$C_2$ alkyl, benzyl, α-methylbenzyl, or phenylethyl, with hydrazine to yield a tautomer of formula IIIa or IIIb of the formula:

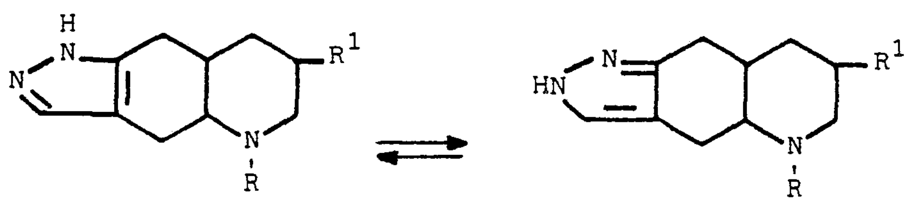

IIIa IIIb

when R and $R^1$ are defined as before.

2. A process of claim 1 in which $R^1$ is H and the ketone starting material is a trans-dl racemate.

3. A process of claim 1 in which the ketone starting material has the 4aR,8aR configuration.

4. The process of claim 1 in which the ketone starting material has the 4aS,8aS configuration.

5. The process which comprises reacting a decahydroquinoline of the formula

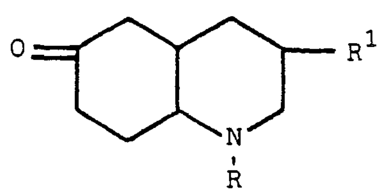

I

wherein R is $C_1$—$C_3$ alkyl, allyl or benzyl and $R^1$ is H or COOZ' wherein Z' is $C_1$—$C_2$ alkyl, benzyl, α-methylbenzyl, or phenylethyl, with a $C_1$—$C_6$ alkyl formate in the presence of base to yield a formyl derivative of the formula

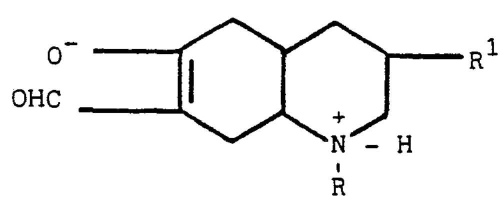

IVb

wherein R and $R^1$ have their previous significance.

6. A process of claim 5 wherein R is n-propyl and $R^1$ is H.

7. A process of claim 5 in which the 1-n-propyl-6-oxodecahydroquinoline starting material is the 4aR,8aR enantiomer.

8. A process of claim 5 in which the 1-n-propyl-6-oxodecahydroquinoline starting material is the 4aS,8aS enantiomer.

9. A process of claim 5 wherein the derivative of formula IVb is further reacted *in situ* with hydrazine to yield a compound of the formula IIIa or IIIb as defined in claim 1.

10. The process of claim 9 where $R^1$ is H and R is n-propyl.

11. The process of claim 9 or 10 wherein the solvent is tetrahydrofuran and the $C_1$—$C_6$ alkylformate is ethyl formate.

12. The process of claim 11 where the compound of formula I is the 4aR,8aR-1-n-propyl-6-oxodecahydroquinoline.

13. The process of claim 11 where the compound of formula I is the 4aS,8aS-1-n-propyl-6-oxodecahydroquinoline.

14. The process of claim 9 which comprises reacting a 4aR,8aR-1-$C_1$—$C_3$ alkyl-6-oxodecahydroquinoline with a $C_1$—$C_6$ alkyl formate in the presence of base to form a 4aR,8aR-1-$C_1$—$C_3$ alkyl-6-oxo-7-formyldecahydroquinoline, and then reacting said formyl compound with hydrazine to form the tautomeric mixture 4aR,8aR-5-$C_1$—$C_3$ alkyl-4,4a,5,6,7,8,8a,9-octahydro-1H(and 2H)pyrazolo[3,4-g]-quinoline.

15. A process of claim 14 in which 4aR,8aR-1-n-propyl-6-oxodecahydroquinoline is converted to the tautomeric mixture 4aR,8aR-5-n-propyl-4,4a,5,6,7,8,8a,9-octahydro-1H(and 2H)pyrazolo[3,4-g]quinoline.

**Patentansprüche für die Vertragsstaaten: BE CH DE FR GB IT LI LU NL SE**

1. Verbindung der Formel IVb

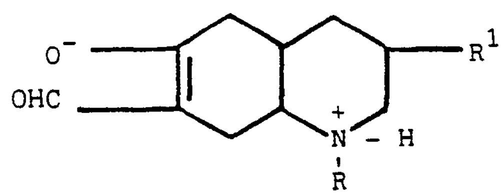

IVb

und deren Tautomere, worin R für $C_1$—$C_3$-Alkyl, Allyl oder Benzyl steht und $R^1$ für Wasserstoff oder COOZ′ steht, wobei Z′ für $C_1$—$C_2$-Alkyl, Benzyl, α-Methylbenzyl oder Phenylethyl steht.

2. Verbindung nach Anspruch 1, dadurch gekennzeichnet, daß R für n-Propyl steht und $R^1$ für Wasserstoff steht, und deren Tautomere.

3. 4aR,8aR-1-n-Propyl-6-oxo-7-formyldecahydrochinolin und deren Tautomere.

4. 4aS,8aS-1-n-Propyl-6-oxo-7-formyldecahydrochinolin und deren Tautomere.

5. trans-dl-1-n-Propyl-6-oxo-7-formyldecahydrochinolin und deren Tautomere.

6. Verfahren zur Herstellung einer Verbindung der Formeln IIIa oder IIIb

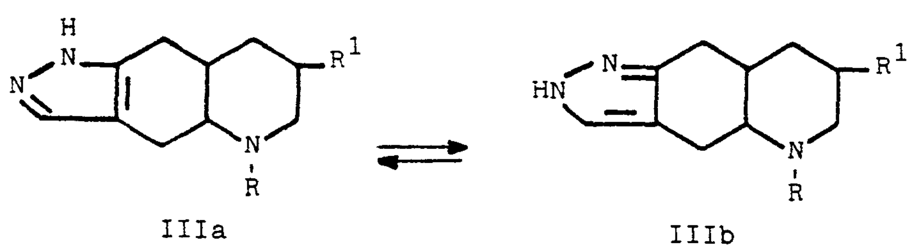

IIIa IIIb

worin R und $R^1$ wie in Anspruch 1 definiert sind, dadurch gekennzeichnet, daß man eine Verbindung der Formel IVb gemäß Anspruch 1 und deren Tautomere mit Hydrazin umsetzt.

7. Verfahren nach Anspruch 6, dadurch gekennzeichnet, daß $R^1$ für H steht und das Ketonausgangsmaterial ein trans-dl-Racemat ist.

8. Verfahren nach Anspruch 6, dadurch gekennzeichnet, daß das Ketonausgangsmaterial die Konfiguration 4aR,8aR hat.

9. Verfahren nach Anspruch 6, dadurch gekennzeichnet, daß das Ketonausgangsmaterial die Konfiguration 4aS,8aS hat.

10. Verfahren zur Herstellung eines Formylderivats der Formel IVb

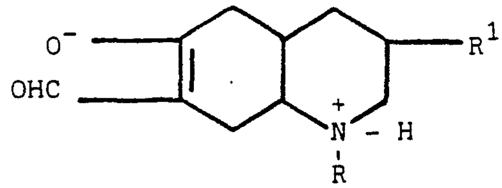

IVb

worin R für $C_1$—$C_3$-Alkyl, Allyl oder Benzyl steht und $R^1$ für H oder COOZ' steht, wobei Z' für $C_1$—$C_2$-Alkyl, Benzyl, α-Methylbenzyl oder Phenylethyl steht, dadurch gekennzeichnet, daß man ein Decahydrochinolin der Formel I

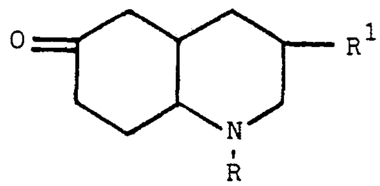

I

worin R und $R^1$ die angegebenen Bedeutungen haben, mit einem $C_1$—$C_6$-Alkylformiat in Gegenwart einer Base umsetzt.

11. Verfahren nach Anspruch 10, dadurch gekennzeichnet, daß R für n-Propyl steht und $R^1$ H steht.

12. Verfahren nach Anspruch 10, dadurch gekennzeichnet, daß das 1-n-Propyl-6-oxodecahydrochinolin-Ausgangsmaterial das 4aR,8aR-Enantiomere ist.

13. Verfahren nach Anspruch 10, dadurch gekennzeichnet, daß das 1-n-Propyl-6-oxodecahydrochinolin-Ausgangsmaterial das 4aS,8aS-Enantiomere ist.

14. Verfahren nach Anspruch 10, dadurch gekennzeichnet, daß man das Derivat der Formel IVb nach dem Verfahren von Anspruch 6 einer weiteren Umsetzung in situ mit Hydrazin unterzieht.

15. Verfahren nach Anspruch 14, dadurch gekennzeichnet, daß $R^1$ für H steht und R für n-Propyl steht.

16. Verfahren nach Anspruch 14 oder 15, dadurch gekennzeichnet, daß das Lösungsmittel Tetrahydrofuran ist und das $C_1$—$C_6$-Alkylformiat Ethylformiat ist.

17. Verfahren nach Anspruch 16, dadurch gekennzeichnet, daß die Verbindung der Formel I das 4aR,8aR-1-n-Propyl-6-oxodecahydrochinolin ist.

18. Verfahren nach Anspruch 14 bis 16, dadurch gekennzeichnet, daß die Verbindung der Formel I das 4aS,8aS-1-n-Propyl-6-oxodecahydrochinolin ist.

19. Verfahren nach Anspruch 14, dadurch gekennzeichnet, daß man 4aR,8aR-1-$C_1$—$C_3$-Alkyl-6-oxodecahydrochinolin mit einem $C_1$—$C_6$-Alkylformiat in Gegenwart einer Base zu einem 4aR,8aR-1-$C_1$—$C_3$-Alkyl-6-oxo-7-formyldecahydrochinolin umsetzt und diese Formylverbindung dann durch Reaktion mit Hydrazin in das tautomere Gemisch von 4aR,8aR-5-$C_1$—$C_3$-Alkyl-4,4a,5,6,7,8,8a,9-octahydro-1H(und 2H)-pyrazol[3,4-g]chinolin überführt.

20. Verfahren nach Anspruch 19, dadurch gekennzeichnet, daß man 4aR,8aR-1-n-Propyl-6-oxodecahydrochinolin in das tautomere Gemisch von 4aR,8aR-5-n-Propyl-4,4a,5,6,7,8,8a,9-octahydro-1H-(und 2H)pyrazol[3,4-g]chinolin überführt.

**Patentansprüche für den Vertragsstaat: AT**

1. Verfahren zur Herstellung eines Tautomeren der Formeln IIIa oder IIIb

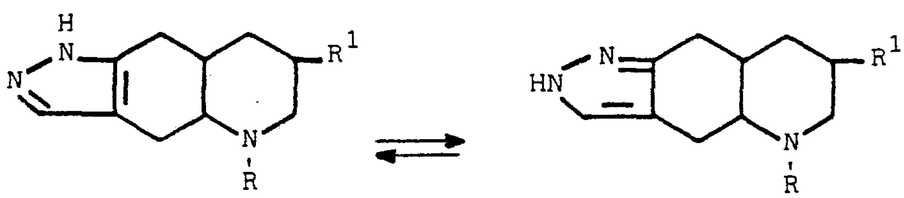

IIIa IIIb

worin R für $C_1$—$C_3$-Alkyl, Allyl oder Benzyl steht und $R^1$ für Wasserstoff oder COOZ' steht, wobei Z' für

$C_1$—$C_2$-Alkyl, Benzyl, α-Methylbenzyl oder Phenylethyl steht, dadurch gekennzeichnet, daß man eine Verbindung der Formel IVb

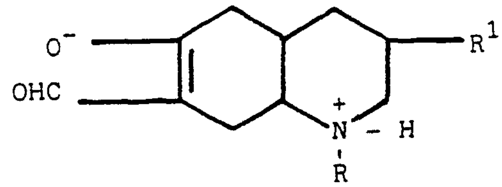

IVb

worin R und $R^1$ wie oben definiert sind, oder ein Tautomeres hiervon mit Hydrazin umsetzt.

2. Verfahren nach Anspruch 1, dadurch gekennzeichnet, daß $R^1$ für H steht und das Ketonausgangsmaterial ein trans-dl-Racemat ist.

3. Verfahren nach Anspruch 1, dadurch gekennzeichnet, daß das Ketonausgangsmaterial die Konfiguration 4aR,8aR hat.

4. Verfahren nach Anspruch 1, dadurch gekennzeichnet, daß das Ketonausgangsmaterial die Konfiguration 4aS,8aS hat.

5. Verfahren zur Herstellung eines Formylderivats der Formel IVb

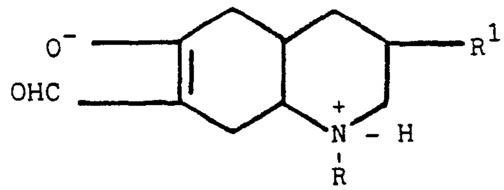

IVb

worin R für $C_1$—$C_3$-Alkyl, Allyl oder Benzyl steht und $R^1$ für H oder COOZ' steht, wobei Z' für $C_1$—$C_2$-Alkyl, Benzyl, α-Methylbenzyl oder Phenylethyl steht, dadurch gekennzeichnet, daß man ein Decahydrochinolin der Formel I

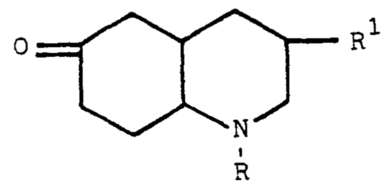

I

worin R und $R^1$ die angegebenen Bedeutungen haben, mit einem $C_1$—$C_6$-Alkylformiat in Gegenwart einer Base umsetzt.

6. Verfahren nach Anspruch 5, dadurch gekennzeichnet, daß R für n-Propyl steht und $R^1$ für H steht.

7. Verfahren nach Anspruch 5, dadurch gekennzeichnet, daß das 1-n-Propyl-6-oxodecahydrochinolin-Ausgangsmaterial das 4aR,8aR-Enantiomere ist.

8. Verfahren nach Anspruch 5, dadurch gekennzeichnet, daß das 1-n-Propyl-6-oxodecahydrochinolin-Ausgangsmaterial das 4aS,8aS-Enantiomere ist.

9. Verfahren nach Anspruch 5, dadurch gekennzeichnet, daß man das Derivat der Formel IVb in situ mit Hydrazin weiter umsetzt und so eine Verbindung der Formel IIIa oder IIIb gemäß Anspruch 1 erhält.

10. Verfahren nach Anspruch 9, dadurch gekennzeichnet, daß $R^1$ für H steht und R für n-Propyl steht.

11. Verfahren nach Anspruch 9 oder 10, dadurch gekennzeichnet, daß das Lösungsmittel Tetrahydrofuran ist und das $C_1$—$C_6$-Alkylformiat Ethylformiat ist.

12. Verfahren nach Anspruch 11, dadurch gekennzeichnet, daß die Verbindung der Formel I das 4aR,8aR-1-n-Propyl-6-oxodecahydrochinolin ist.

13. Verfahren nach Anspruch 11, dadurch gekennzeichnet, daß die Verbindung der Formel I das 4aS,8aS-1-n-Propyl-6-oxodecahydrochinolin ist.

14. Verfahren nach Anspruch 9, dadurch gekennzeichnet, daß man 4aR,8aR-1-$C_1$—$C_3$-Alkyl-6-oxodecahydrochinolin mit einem $C_1$—$C_6$-Alkylformiat in Gegenwart einer Base zu einem 4aR,8aR-1-$C_1$—$C_3$-Alkyl-6-oxo-7-formyldecahydrochinolin umsetzt und diese Formylverbindung dann durch Reaktion mit Hydrazin in das tautomere Gemisch von 4aR,8aR-5-$C_1$—$C_3$-Alkyl-4,4a,5,6,7,8,8a,9-octahydro-1H(und 2H)-pyrazol[3,4-g]chinolin überführt.

15. Verfahren nach Anspruch 14, dadurch gekennzeichnet, daß man 4aR,8aR-1-n-Propyl-6-oxodecahydrochinolin in das tautomere Gemisch von 4aR,8aR-5-n-Propyl-4,4a,5,6,7,8,8a,9-octahydro-1H-(und 2H)pyrazol[3,4-g]chinolin überführt.

**Revendications pour les Etats contractants: BE CH DE FR GB IT LI LU NL SE**

1. Composé de formule:

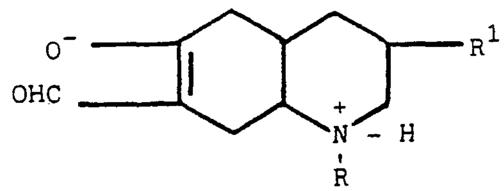

IVb

et ses tautomères, formule dans laquelle R représente un groupe alkyle en $C_1$—$C_3$, un groupe allyle ou un groupe benzyle, $R^1$ représente un atome d'hydrogène ou COOZ', tandis que Z' représente un groupe alkyle en $C_1$—$C_2$, un groupe benzyle, un groupe α-méthylbenzyle ou un groupe phényléthyle.

2. Composé selon la revendication 1, dans lequel R est un groupe n-propyle et $R^1$ est un atome d'hydrogène, de même que ses tautomères.

3. La 4aR,8aR-1-n-propyl-6-oxo-7-formyldécahydroquinoléine et ses tautomères.

4. La 4aS,8aS-1-n-propyl-6-oxo-7-formyldécahydroquinoléine et ses tautomères.

5. La trans-dl-1-n-propyl-6-oxo-7-formyldécahydroquinoléine et ses tautomères.

6. Procédé en vue de faire réagir un composé de formule IVb comme défini dans la revendication 1 et ses tautomères avec l'hydrazine pour obtenir un tautomère de formule IIIa ou IIIb:

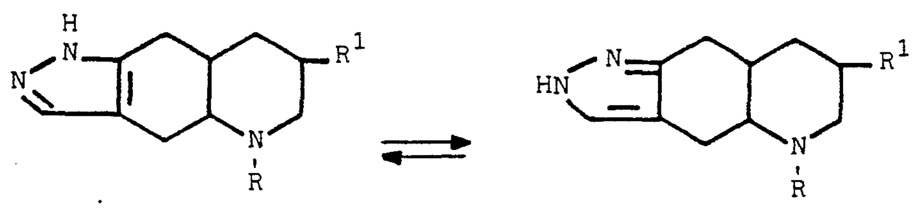

IIIa IIIb

lorsque R et $R^1$ ont les significations définies dans la revendication 1.

7. Procédé selon la revendication 6, dans lequel $R^1$ représente un atome d'hydrogène, tandis que la matière de départ cétonique est un racémate trans-dl.

8. Procédé selon la revendication 6, dans lequel la matière de départ cétonique a la configuration 4aR,8aR.

9. Procédé selon la revendication 6, dans lequel la matière de départ cétonique a la configuration 4aS,8aS.

10. Procédé consistant à faire réagir une décahydroquinoléine de formule:

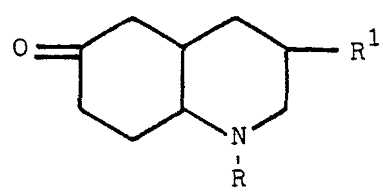

I

dans laquelle R représente un groupe alkyle en $C_1$—$C_3$, un groupe allyle ou un groupe benzyle, tandis que $R^1$ représente un atome d'hydrogène ou COOZ' où Z' représente un groupe alkyle en $C_1$—$C_2$, un groupe benzyle, un groupe α-méthylbenzyle ou un groupe phényléthyle, avec un formiate d'alkyle en $C_1$—$C_6$ en présence d'une base pour obtenir un dérivé formyle de formule:

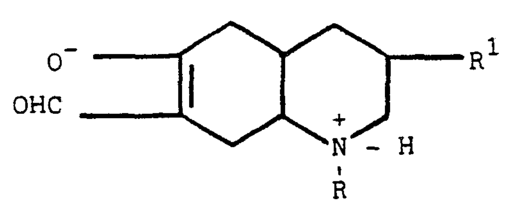

IVb

dans laquelle R et $R^1$ ont les significations définies précédemment.

11. Procédé selon la revendication 10, dans lequel R est un groupe n-propyle et $R^1$ est un atome d'hydrogène.

12. Procédé selon la revendication 10, dans lequel la matière de départ 1-n-propyl-6-oxo-décahydroquinoléine est l-énantiomère 4aR,8aR.

13. Procédé selon la revendication 10, dans lequel la matière de départ 1-n-propyl-6-oxo-décahydroquinoléine est l'énantiomère 4aS,8aS.

14. Procédé selon la revendication 10, dans lequel on soumet le dérivé de formule IVb à une réaction complémentaire *in situ* avec l'hydrazine conformément au procédé de la revendication 6.

15. Procédé selon la revendication 14, dans lequel $R^1$ représente un atome d'hydrogène et R représente un groupe n-propyle.

16. Procédé selon la revendication 14 ou 15, dans lequel le solvant est le tétrahydrofuranne, tandis que le formiate d'alkyle en $C_1$—$C_6$ est le formiate d'éthyle.

17. Procédé selon la revendication 16, dans lequel le composé de formule I est la 4aR,8aR-1-n-propyl-6-oxodécahydroquinoléine.

18. Procédé selon les revendications 14 à 16, dans lequel le composé de formule I est la 4aS,8aS-1-n-propyl-6-oxodécahydroquinoléine.

19. Procédé selon la revendication 14, caractérisé en ce qu'il consiste à faire réagir une 4aR,8aR-1-alkyl en $C_1$—$C_3$-6-oxodécahydroquinoléine avec un formiate d'alkyle en $C_1$—$C_6$ en présence d'une base pour former une 4aR,8aR-1-alkyl en $C_1$—$C_3$-6-oxo-7-formyldécahydroquinoléine, puis faire réagir ce composé formyle avec l'hydrazine pour former le mélange tautomère 4aR,8aR-5-alkyl en $C_1$—$C_3$-4,4a,5,6,7,8,8a,9-octahydro-1H(et 2H)pyrazolo[3,4-g]quinoléine.

20. Procédé selon la revendication 19, dans lequel la 4aR,8aR-1-n-propyl-6-oxodécahydroquinoléine est convertie en mélange tautomère 4aR,8aR-5-n-propyl-4,4a,5,6,7,8,8a,9-octahydro-1H(et 2H)pyrazolo[3,4-g]quinoléine.

**Revendications pour l'Etat contractant: AT**

1. Procédé en vue de faire réagir un composé de formule:

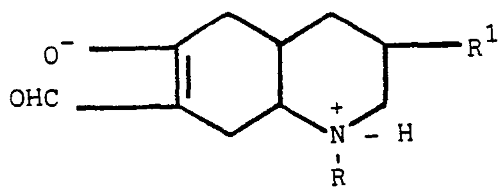

IVb

et ses tautomères, formule dans laquelle R représente un groupe alkyle en $C_1$—$C_3$, un groupe allyle ou un groupe benzyle, $R^1$ représente un atome d'hydrogène ou COOZ' et Z' représente un groupe alkyle en $C_1$—$C_2$, un groupe benzyle, un groupe α-méthylbenzyle ou un groupe phényléthyle, avec l'hydrazine pour obtenir un tautomère de formule IIIa ou IIIb:

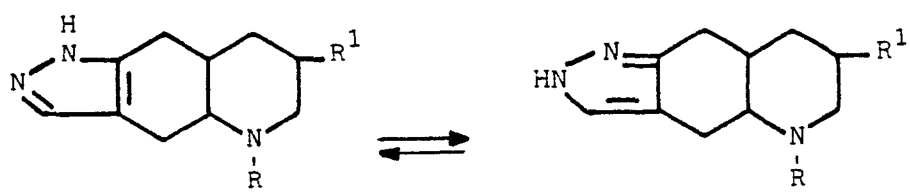

IIIa IIIb

lorsque R et $R^1$ ont les significations définies ci-dessus.

2. Procédé selon la revendication 1, dans lequel $R^1$ est un atome d'hydrogène, tandis que la matière de départe cétonique est un racémate trans-dl.

3. Procédé selon la revendication 1, dans lequel la matière de départ cétonique a la configuration 4aR,8aR.

4. Procédé selon la revendication 1, dans lequel la matière de départ cétonique a la configuration 4aS,8aS.

5. Procédé consistant à faire réagir une décahydroquinoléine de formule:

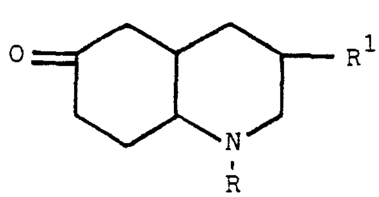

I

dans laquelle R représente un groupe alkyle en $C_1$—$C_3$, un groupe allyle ou un groupe benzyle et $R^1$ représente un atome d'hydrogène ou COOZ' où Z' représente un groupe alkyle en $C_1$—$C_2$, un groupe benzyle, un groupe α-méthylbenzyle ou un groupe phényléthyle, avec un formiate d'alkyle en $C_1$—$C_6$ en présence d'une base pour obtenir un dérivé formyle de formule:

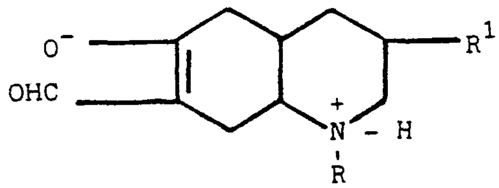

IVb

dans laquelle R et $R^1$ ont les significations définies précédemment.

6. Procédé selon la revendication 5, dans lequel R est un groupe n-propyle et $R^1$ représente un atome d'hydrogène.

7. Procédé selon la revendication 5, dans lequel la matière de départ 1-n-propyl-6-oxo-décahydroquinoléine est l-énantiomère 4aR,8aR.

8. Procédé selon la revendication 5, dans lequel la matière de départ 1-n-propyl-6-oxo-décahydroquinoléine est l'énantiomère 4aS,8aS.

9. Procédé selon la revendication 5, dans lequel on soumet le dérivé de formule IVb à une rèaction complémentaire *in situ* avec l'hydrazine pour obtenir un composé de formule IIIa ou IIIb comme défini dans la revendication 1.

10. Procédé selon la revendication 9, dans lequel $R^1$ représente un atome d'hydrogène et R représente un groupe n-propyle.

11. Procédé selon la revendication 9 ou 10, dans lequel le solvant est le tétrahydrofuranne, tandis que le formiate d'alkyle en $C_1$—$C_6$ est le formiate d'éthyle.

12. Procédé selon la revendication 11, dans lequel le composé de formule I est la 4aR,8aR-1-n-propyl-6-oxodécahydroquinoléine.

13. Procédé selon la revendication 11, dans lequel le composé de formule I est la 4aS,8aS-1-n-propyl-6-oxodécahydroquinoléine.

14. Procédé selon la revendication 9, caractérisé en ce qu'il consiste à faire réagir une 4aR,8aR-1-alkyl en $C_1$—$C_3$-6-oxodécahydroquinoléine avec un formiate d'alkyle en $C_1$—$C_6$ en présence d'une base pour former une 4aR,8aR-1-alkyl en $C_1$—$C_3$-6-oxo-7-formyldécahydroquinoléine, puis faire réagir ce composé formyle avec l'hydrazine pour former le mélange tautomère 4aR,8aR-5-alkyl en $C_1$—$C_3$-4,4a,5,6,7,8,8a,9-octahydro-1H(et 2H)pyrazolo[3,4-g]quinoléine.

15. Procédé selon la revendication 14, dans lequel la 4aR,8aR-1-n-propyl-6-oxodécahydroquinoléine est convertie en mélange tautomère 4aR,8aR-5-n-propyl-4,4a,5,6,7,8,8a,9-octahydro-1H(et 2H)pyrazolo[3,4-g]quinoléine.